(19) Europäisches Patentamt
European Patent Office
Office européen des brevets

(11) **EP 4 722 716 A1**

(12) **EUROPEAN PATENT APPLICATION**

(43) Date of publication:
08.04.2026 Bulletin 2026/15

(21) Application number: **25205545.4**

(22) Date of filing: **30.09.2025**

(51) International Patent Classification (IPC):
**G01N 33/18** (2006.01)     **G06N 20/00** (2019.01)

(52) Cooperative Patent Classification (CPC):
**G01N 33/1846; G06N 3/08; G06N 20/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **04.10.2024  US 202418907222**

(71) Applicant: **Mettler-Toledo Thornton, Inc.**
**Billerica MA 01821 (US)**

(72) Inventors:
• **Theriault, Robert**
  **Tyngsborough, MA 01821 (US)**
• **Reijnders, Anjan**
  **Groton, MA 01450 (US)**

(74) Representative: **Mettler-Toledo**
**IP Department**
**Im Langacher 44**
**8606 Greifensee (CH)**

(54) **SYSTEMS AND METHODS PROVIDING ARTIFICIAL INTELLIGENCE ANALYSIS OF TOC SENSOR DATA**

(57)     Systems and methods for providing artificial intelligence analysis of total organic carbon ("TOC") sensor data are provided. A TOC measurement device includes a fluid containing portion for holding a sample fluid and sensor(s) and an oxidation device at the fluid containing portion. A controller operates the oxidation device to cause the sample fluid at the fluid containing portion to experience differing levels of exposure to the oxidation device over a time period, receives measurements from the sensor(s) over the time period, each taken at a different time such that each of the measurements reflect a different exposure level of the sample fluid from the oxidation device, analyzes the measurements, including by applying a deterministic model, to determine an initial TOC measurement for the sample fluid, and applies an artificial intelligence module to the initial TOC measurement to determine an error compensated TOC measurement.

FIG. 1A

**Description**

**TECHNICAL FIELD**

Exemplary embodiments relate generally to systems and methods providing artificial intelligence ("AI") analysis of total organic carbon ("TOC") sensor data.

**BACKGROUND AND SUMMARY OF THE INVENTION**

Various devices are known for analyzing TOC of fluids. Such devices may be important for measuring purity of water, which may be particularly useful for sensitive applications such as pharmaceutical applications, by way of non-limiting example. A certain level of sensitivity to particular compounds may be required to meet various regulatory or scientific standards (e.g., requirements). Some TOC sensor devices provide ongoing measurements for use with a continuously flowing system. Typically, such TOC sensor devices are fluidly connected to a larger fluid system and are configured to take (continuously, periodically, selectively, etc.) a sample of the flow in the larger fluid system for TOC analysis (hereinafter also "testing").

Some TOC sensor devices use conductivity sensors to measure conductivity of a sample fluid. Certain known approaches to measuring TOC involve single data point analysis by taking a first conductivity measurement of the sample fluid, at least partially oxidizing the sample fluid, and taking a second conductivity measurement. At least in theory, the first measurement represents inorganic carbon (IC) in the sample fluid and the second measurement represents total carbon (TC). A difference in the conductivity measurements is used to derive a TOC measurement for the sample fluid, thereby representing the TOC measurement for the larger system. Scientific instruments, including TOC sensor devices, are subject to certain levels of error in measurement based on the unpredictability and randomness of various factors, such as flow rate of fluid measured, gas bubble interference within fluid, ultraviolet (UV) light exposure, temperature of fluid, and the like. Additionally, such measurements may be subject to a certain level of error based on the randomly occurring presence of interfering species in the sample fluid. Such interfering species include compounds within the sample fluid, at any point during the oxidation process, which are conductive (e.g., weak organic acids, strong inorganic acids, and conductive reactants), but do not necessarily oxidize completely to $CO_2$. These interfering species may be non-conductive compounds before oxidation which may chemically transform, generally through a number of intermediary conversions, into conductive compounds during the oxidation process. Examples of such conversions are provided below, by way of non-limiting example:

$$C_xH_yO_z + UV \text{ (e.g., 184 and 254nm)} \rightarrow CO_2 + H_2O \leftrightarrow H_2CO_3 \leftrightarrow H^+ + HCO_3^- \qquad \text{Example Conversion 1 (non-interfering)}$$

$$CHCl_3 + UV \text{ (e.g., 184 and 254nm)} \rightarrow CO_2 + H_2O + HCl \leftrightarrow H_2CO_3 + HCl \leftrightarrow H^+ + Cl^- + HCO_3^-$$
Example Conversion 2 (interfering)

As such, these interfering species may be reflected in the second measurement, but not in the first measurement, thereby resulting in a too high TOC measurement.

Known approaches to minimizing error from the presence of interfering species generally involve separating carbon dioxide ($CO_2$) from the oxidized sample fluid (and thus also the interfering species). Such separation may be achieved by way of membrane separation (see e.g., US Pat. No. 5,132,094) or sparging (see e.g., US Pat. No. 9,791,430), for example, and the separated $CO_2$ may subsequently be measured with a sensor, such as a conductivity sensor, or a nondispersive infrared (NDIR) sensor, respectively. These approaches take time and are complicated to implement, increasing costs and necessary calibration efforts. Furthermore, taking such TOC measurements may take additional time, which may take time away from producing the desired product. However, when measuring TOC with single data point analysis or other known approaches, especially without separating $CO_2$, errors can persist in these known approaches due to the unpredictability and randomness of many variables, but not necessarily limited to, sample fluid composition, flow rate variability, temperature variability, UV dosage variability, combinations thereof, or the like. A more accurate error compensation approach, particularly without the need to separate $CO_2$, which is preferably also time efficient, is therefore desirable.

The inventive system and method for total organic carbon ("TOC") analysis with an active, such as continuous, flow device according to the claims overcomes these deficiencies.

The inventive method for providing artificial intelligence analysis of total organic carbon ("TOC") sensor data, said method comprising:

operating a TOC measurement device to cause differing exposure of a fluid at a fluid containing portion of the TOC measurement device from an oxidation device of the TOC measurement device over time;

receiving, at a controller, a plurality of measurements from one or more sensors of the TOC measurement device at the fluid containing portion of the TOC measurement device over time such that each of the plurality of measurements reflect a different exposure level of the fluid to the oxidation device;

analyzing, by way of the controller, the plurality of measurements, including by applying a deterministic model to the plurality of measurements, to determine an initial TOC measurement for the fluid, and by applying an artificial intelligence module to the initial TOC measurement to determine an error compensated TOC measurement.

The inventive system for providing artificial intelligence analysis of total organic carbon ("TOC") sensor data, said system comprising:

a TOC measurement device comprising:

a fluid containing portion for holding a fluid;
one or more sensors at the fluid containing portion; and
an oxidation device at the fluid containing portion; and

a controller comprising one or more non-transitory electronic storage devices comprising software instructions, which when executed, configure one or more processors to:

operate the oxidation device to cause the fluid at the fluid containing portion to experience differing levels of exposure to the oxidation device over a time period;
receive a plurality of measurements from the one or more sensors over the time period, each taken at a different time such that each of the measurements reflect a different exposure level of the fluid sample from the oxidation device;
analyze the plurality of measurements, including by applying a deterministic model, to determine an initial TOC measurement for the fluid; and
apply an artificial intelligence module to the initial TOC measurement to determine an error compensated TOC measurement.

The invention further relates to a system according to claim 15.

Preferred embodiments of the aspects of the present invention are stated in the corresponding dependent claims and are described below.

Inventive systems and methods are disclosed which provide AI analysis of TOC sensor data. The AI knowledge used for TOC analysis relates to, by of way non-limiting example, TOC error compensation, supervised learning routines, intelligent control, combinations thereof, or the like. The inventive systems and methods harness AI knowledge used for TOC analysis by first collecting multiple data points for oxidation analysis, such as by a TOC measurement device. The multiple data points may represent various oxidation conditions and/or exposure times of a sample to an oxidation device (e.g., UV lamp, reagent, combustion, electrochemical oxidation, etc.). The multiple data points may be acquired from sensor(s) of the TOC measurement device. The data may be reduced. For example, without limitation, in the case of conductivity measurements, the first conductivity measurement for a sample may be subtracted from the second conductivity measurement. A deterministic model that assumes the absence of unpredictability and randomness may be applied to the measurements, such as to arrive at an initial TOC measurement. For example, without limitation, the deterministic model may utilize known curve fitting, regression/extrapolation techniques to predict the initial TOC measurement at a future or particular time, and/or a multi-parameter, weighted, regression analysis to essentially fit a curve to the multiple data points, where each parameter represents a quantified state of the oxidation curve.

A stochastic analysis module may be applied to the initial TOC measurement to determine an estimated error, which may be applied to the initial TOC measurement to determine an error compensated TOC measurement for output. The stochastic analysis module may include an artificial intelligence module, which may include a stochastic error model, and/or expert knowledge system. The expert knowledge system may comprise one or more databases with rules. In exemplary embodiments, without limitation, the artificial intelligence module may be developed through provision of training data sets that are manually classified and represent certain controlled conditions, such as samples with known compositions, at known temperatures, at known flow rates, combinations thereof, or the like. The artificial intelligence module may include a stochastic error model, which may be configured to develop, may comprise, and/or may utilize a multi-dimensional error analysis hypersurface, such as, but not necessarily limited to a three-dimensional error analysis mesh, representing the parameters of the deterministic model and error, such as absolute error. The artificial intelligence module may utilize an N dimension tensor to store the fitting parameters from the regression fit of the deterministic model, along with other analytical results, where $N >= 0$. By applying the parameters to the multi-dimensional error analysis hypersurface, a corresponding error (e.g., absolute, relative) may be provided which is applied to the initial TOC measurement to arrive at the error compensated TOC measurement. A self-learning feedback loop may be provided to accommodate manual user feedback regarding the error compensated TOC measurement and/or user provided training data sets and/or rules, which may be incorporated into the artificial intelligence module and/or the expert knowledge systems. The initial TOC measurement and/or error compensated TOC measurement may be adjusted, such as by known compensation and/or conversion techniques, such as to arrive at a ppbC carbon or other measurement for output. The system may be configured to provide alerts, such as electronic notification, upon analysis results, including TOC measurements meeting certain predetermined rules (e.g., limits, changes, indications of particular compounds present, combinations thereof, or the like), which may be user defined. Intelligent control over an associated system or components thereof may be provided.

In exemplary embodiments, without limitation, a system for providing artificial intelligence analysis of TOC sensor data includes a TOC measurement device comprising: a fluid containing portion for holding a fluid, one or more sensors at the fluid containing portion, and an oxidation device at the fluid containing portion.

A controller comprising one or more non-transitory electronic storage devices comprising software instructions, which when executed, configure one or more processors to: operate the oxidation device to cause the fluid at the fluid containing portion to experience differing levels of exposure to the oxidation device over a time period; receive a plurality of measurements from the one or more sensors over the time period, each taken at a different time such that each of the measurements reflect a different exposure level of the fluid sample from the oxidation device; analyze the plurality of measurements, including by applying a deterministic model, to determine an initial TOC measurement for the fluid; and apply an artificial intelligence module to the initial TOC measurement to determine an error compensated TOC measurement.

The error compensated TOC measurement may be provided in, or converted to, ppbC or another format.

The deterministic model may comprise a multi-parameter, weighted, regression analysis which fits the plurality of measurements to a curve.

The artificial intelligence module may utilize a multi-dimension tensor to store regression analysis parameters from the deterministic model.

The artificial intelligence module may be developed with manually classified training data sets with controlled specification, including fluid sample composition which develop a multi-dimensional error analysis hypersurface representing the parameters and error. The expert knowledge system may, optionally, be updated with the same or different training data sets.

The artificial intelligence module may comprise a stochastic error model which applies the multi-dimensional error analysis hypersurface to the parameters of the deterministic model and adjusts the initial TOC measurement to the error compensated TOC measurement based on the resulting error value.

The system may include an expert knowledge system comprising one or more databases comprising rules. The artificial intelligence module or the deterministic model may be configured to query the expert knowledge system prior to determining the error compensated TOC measurement. The rules may comprise at least one rule for classification of at least one chemical type.

The stochastic error model may include a self-learning stochastic feedback loop. The controller may include additional software instructions stored at the one or more non-transitory electronic storage devices, which when executed, configures the one or more processors to: receive data indicating verification or denial of the error compensated TOC measurement; and update the stochastic error model in accordance with the data indicating verification or denial of the error compensated TOC measurement as part of the self-learning stochastic feedback loop.

The TOC measurement device may include an active flow device.

The fluid containing portion of the TOC measurement device may include a flow channel. The oxidation device of the TOC measurement device may include an ultraviolet ("UV") lamp. The TOC measurement device may include a flow control device configured to control a flow rate of the fluid through the flow channel. The controller may comprise additional software instructions stored at the one or more non-transitory electronic storage devices, which when executed, configures the one or more processors to: alter the flow rate of the fluid through the flow channel in a cyclical fashion while the plurality of measurements is taken and the oxidation device is active.

The flow channel of the TOC measurement device may be shaped into a coil about the UV lamp.

The TOC measurement device may include a batch processing device, the one or more sensors may include conductivity sensors, the fluid containing portion may include a fluid chamber configured to statically hold the fluid for a period of time, the oxidation device of the TOC measurement device may include at least one of: an ultraviolent ("UV") lamp, a reagent dispenser, a heating element, a catalyst device, and an electrochemical device. The controller may include additional software instructions stored at the one or more non-transitory electronic storage devices, which when executed, configures the one or more processors to: take the plurality of measurements at different times.

The controller of the system may be remote from the TOC measurement device and in electronic communication with at least one component of an end-user system to which the TOC measurement device is fluidly connected. The controller may include additional software instructions stored at the one or more non-transitory electronic storage devices, which when executed, configures the one or more processors to: command operation of at least one component of the end-user system.

In exemplary embodiments, without limitation, a method for providing artificial intelligence analysis of TOC sensor data includes: operating a TOC measurement device to cause differing exposure of a fluid at a fluid containing portion of the TOC measurement device from an oxidation device of the TOC measurement device over time; receiving, at a controller, a plurality of measurements from one or more sensors of the TOC measurement device at the fluid containing portion of the TOC measurement device over time such that each of the plurality of measurements reflect a different exposure level of the fluid to the oxidation device; analyzing, by way of the controller, the plurality of measurements, including by applying a deterministic model to the plurality of measurements, to determine an initial TOC measurement for the fluid, and by applying an artificial intelligence module to the initial TOC measurement to determine an error compensated TOC measurement.

The method may further include developing the artificial intelligence module with manually classified training data sets with controlled specification, including fluid sample composition which develop a multi-dimensional error analysis hypersurface representing multiple parameters and error, wherein the deterministic model comprises the multiple parameters as part of a weighted, regression analysis which fits the plurality of measurements to a curve; and applying, by way of the artificial intelligence module and the controller, the multi-dimensional error analysis hypersurface to the parameters of the deterministic model, and adjusting, by way of the controller, the initial TOC measurement to the error compensated TOC measurement based on the resulting error value.

The method may further include querying, by way of the controller, an expert knowledge system prior to determining the error compensated TOC measurement, said expert knowledge system comprising one or more databases comprising rules, including at least one rule for classifying at least one chemical type.

The method may further include receiving, by way of the controller, data indicating verification or denial of the error compensated TOC measurement; and updating, by way of the controller, the multi-dimensional error analysis hypersurface of the stochastic error model in accordance with the data indicating verification or denial of the error compensated TOC measurement as part of the self-learning stochastic feedback loop.

The error compensated TOC measurement may be provided in, or converted into, a ppbC measurement.

The TOC measurement device may include an active flow device, the fluid containing portion may include a sample fluid passageway, and the step of operating the TOC measurement device to cause differing exposure of the fluid at the fluid containing portion of the TOC measurement device to the oxidation device of the TOC measurement device over time may include causing a cyclic, active flow rate at the sample fluid passageway over time.

In exemplary embodiments, without limitation, a system for providing artificial intelligence analysis of TOC sensor data, includes a TOC measurement device comprising: a fluid sample passageway configured to hold a fluid, one or more sensors located at the fluid containing portion, an oxidation device at the fluid containing portion, and a flow control device located at the fluid containing portion. A controller includes one or more non-transitory electronic storage devices comprising software instructions, which when executed, configure one or more processors to: operate the flow control device to cause a cyclic, non-zero, active flow rate of the fluid through the fluid sample passageway over time; receive a plurality of measurements from the one or more sensors, where each of the plurality of measurements are taken at a different time such that each of the measurements reflect a different oxidation level of the fluid; fit the plurality of measurements to a curve to generate a regression curve for the fluid; apply a deterministic model to the plurality of measurements to determine an initial TOC measurement for the fluid; and apply an artificial intelligence module to the initial TOC measurement to determine an error compensated TOC measurement.

Further features and advantages of the systems and methods disclosed herein, as well as the structure and operation of various aspects of the present disclosure, are described in detail below with reference to the accompanying figures.

**BRIEF DESCRIPTION OF THE DRAWINGS**

In addition to the features mentioned above, other aspects of the present invention will be readily apparent from the following descriptions of the drawings and exemplary embodiments, wherein like reference numerals across the several views refer to identical, similar, or equivalent features, and wherein:

**FIGURE 1A** is a schematic view of an exemplary active flow TOC analysis device and related system;
**FIGURE 1B** is a schematic view of another exemplary TOC analysis device;
**FIGURE 2** is a graph with an exemplary oxidation curve indicating exemplary measurement error possibilities;
**FIGURE 3** is a graph with various exemplary oxidation curves;
**FIGURE 4** is a flow chart with an exemplary method for operating the TOC analysis device of figure 1A;
**FIGURE 5** is a graph with an exemplary oxidation curve overlaid with multiple exemplary data points, such as gathered and/or processed using the TOC analysis device of figure 1A and/or method of figure 4;

**FIGURE 6A** is a graph demonstrating, in an exemplary fashion, how the TOC analysis device of figure 1A and/or method of figure 4 generate the oxidation curve data of figure 5 in an exemplary fashion;

**FIGURE 6B** is another graph further demonstrating, in an exemplary fashion, how the TOC analysis device of figure 1A and/or method of figure 4 generate the oxidation curve data of figure 5 in an exemplary fashion;

**FIGURE 6C** is another graph further demonstrating, in an exemplary fashion, how the TOC analysis device of figure 1A and/or method of figure 4 generate the oxidation curve data of figure 5 in an exemplary fashion;

**FIGURE 7A** is a schematic illustration of multiple data points that may be processed using the TOC analysis device of figure 1A and/or method of figure 4;

**FIGURE 7B** is an exemplary graphical representation of multiple data points from the measurements of figure 7A;

**FIGURE 8** is a schematic overview and flow chart of an exemplary enhanced data processing method for multiple data points, such as obtained by the TOC analysis device of figure 1A and/or method of figure 4; and

**FIGURE 9** is a schematic view of an exemplary TOC analysis system for the TOC analysis device of figure 1A and/or the methods of figures 4 and/or 8.

## DETAILED DESCRIPTION OF EXEMPLARY EMBODIMENT(S)

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope and spirit of the present invention. In addition, descriptions of well-known functions and constructions are omitted for clarity and conciseness.

Embodiments of the invention are described herein with reference to illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

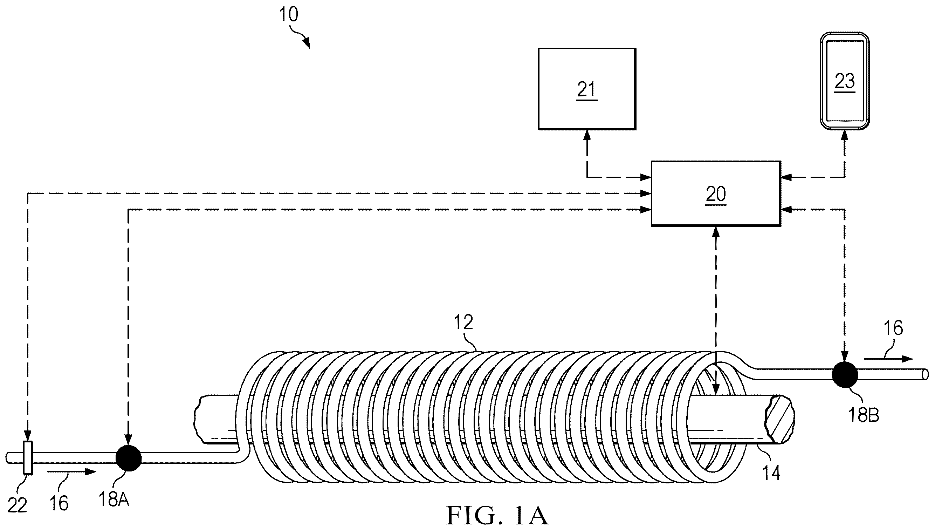

**FIGURE 1A** illustrates an exemplary TOC device 10. The TOC device 10 may be an active flow analysis type device. The TOC device 10 may comprise a sample fluid passageway 12 and one or more oxidation devices, such as but not necessarily limited to, a light source 14, such as, but not necessarily limited to, an ultraviolet (UV) lamp. The light source 14 may be configured to, when activated, cause ultraviolet light at various wavelengths, such as at some wavelength between about 100nm and 400nm, such as 172nm, 185nm, and/or 254nm, by way of non-limiting example. When activated, the light source 14 may irradiate a flow of sample fluid 16 within the sample fluid passageway 12. The sample fluid passageway 12 may include any size or shape enclosed passageway (e.g., tube, pipe, duct, or the like) for a liquid and/or gas, such as purified water.

The light source 14 may be positioned sufficiently proximate to at least a first portion of the sample fluid passageway 12 to permit irradiation of the sample fluid 16 within at least the first portion of the sample fluid passageway 12 when the light source 14 is activated. In exemplary embodiments, without limitation, at least the first portion of the sample fluid passageway 12 may be provided in a coil or other shape about some or all of the light source 14, such as to keep the sample fluid 16 within a sufficient distance of the light source 14 to expose the sample fluid 16, thereby causing oxidation of any oxidizing species therein. Various size, shape, type, and/or number of sample fluid passageways 12 and/or light sources 14 may be utilized. At least the first portion of the sample fluid passageway 12 may comprise a UV transparent or translucent material, such as quartz by way of non-limiting example, to facilitate such oxidation from the light source 14.

Exposure of organic compounds in the sample fluid 16 to the UV light may result in oxidation, such as demonstrated by example conversion1 and/or 2, without limitation.

Conductivity sensors 18 may be provided along the sample fluid passageway 12. In exemplary embodiments, without limitation, a first conductivity sensor 18A is provided at an entrance to a first portion of the sample fluid passageway 12 and a second conductivity sensor 18B is provided at an exit of the first portion of the sample fluid passageway 12. Other number, location, and/or type of such sensors 18 may be utilized within the sample fluid passageway 12. While a coil shape is sometimes shown and/or discussed, other arrangements and/or number of the sample fluid passageway 12 may be utilized, such as but not limited to, a shell and tube type arrangement (e.g., light sources(s) 14 surrounded by sample fluid passageway 12), light sources(s) 14 positioned outside and/or along the sample fluid passageway(s) 12, which may be straight, curved, in a zig-zag pattern, combinations thereof, or the like.

The TOC device 10 may comprise one or more flow control devices 22. The flow control device 22 may be positioned along the sample fluid passageway(s) 12 and may be electronically controlled or otherwise actuated to adjust a flow rate of the sample fluid 16 within the sample fluid passageway(s) 12. The flow control devices 22 may include valves, pumps, gas pressure devices, gravity feed devices, combinations thereof, or the like. The flow control device 22 may be positioned at an entrance to the first portion of the sample fluid passageway 12, for example. However, different number, type, and/or location of such flow control devices 22 may be utilized. For example, without limitation, the flow control devices 22 may be provided at the entrance and/or exit to the sample fluid passageway 12, along the sample fluid passageway 12, before or after the sample fluid passageway 12, combinations thereof, or the like.

The conductivity sensors 18, light source 14, flow control device 22, and/or other components of the TOC device 10 may be in electronic communication (wired and/or wireless) with a controller 20. The controller 20 may be part of the TOC device 10 or separate therefrom. The controller 20 may be configured to operationally control such components, such as by way of electronically issued commands to the same, and/or may be configured to receive multiple data points from the same, such as regarding operational status or data measurements.

In exemplary embodiments, without limitation, the controller 20 is configured to receive measurements from the sensors 18 while a flow of the sample fluid 16 through the sample fluid passageway 12 is active, and, preferably, while the light source 14 is also active. The measurements from the sensors 18 may be unconverted or may be converted to other units, or other formats, for further analysis and/or processing. A difference between the measurements from the first and second conductivity sensors 18A and 18B may be used by the controller 20 to determine a TOC measurement. For example, without limitation, the first conductivity sensor 18A may take a first conductivity measurement of the sample fluid 16 at the sample fluid passageway 12, which may be designated C1, and which may represent an inorganic carbon (IC) measurement of the sample. As the sample fluid 16 within the sample fluid passageway 12 is oxidized, such as by exposure, or continued exposure, to the light source 14, the second conductivity sensor 18B may take a second conductivity measurement, which may be designated C2, and which may represent a total carbon (TC) measurement of the sample fluid 16. The total organic carbon (TOC) may be calculated as TC - IC. In exemplary embodiments, without limitation, the TOC is calculated from a difference between the first and second conductivity measurements, which may be designated $\Delta C$ and/or referred to herein as a delta conductivity measurement, in accordance with the following equation:

$$TOC = function\ (\Delta C),\ where\ \Delta C = C2 - C1$$

## Equation 1

Each of the delta conductivity measurements ($\Delta C$) may be processed to provide an initial TOC measurement, such as by way of one or more compensation adjustments (e.g., for sample fluid 16 temperature) and/or into a different form (e.g., ppbC). As further described herein, each of the delta conductivity measurements ($\Delta C$), the first or second conductivity measurement of converted or unconverted units, and/or TOC measurements may be fit to a curve. As further described herein, these initial measurements may be error compensated, such as through various AI-utilizing techniques, to arrive at an error compensated TOC measurement, such as in ppbC.

Alternatively, or additionally, a single measurement from the second conductivity sensor 18B (which may be the only conductivity sensor 18 for the TOC device 10) may be used to generate some or all of the multiple data points, such as where the TOC device 10 is a batch processing device or an active flow device. In the case of an active flow device, an oxidation curve could be derived in certain situations from data from the conductivity sensor 18B only, by way of non-limiting example.

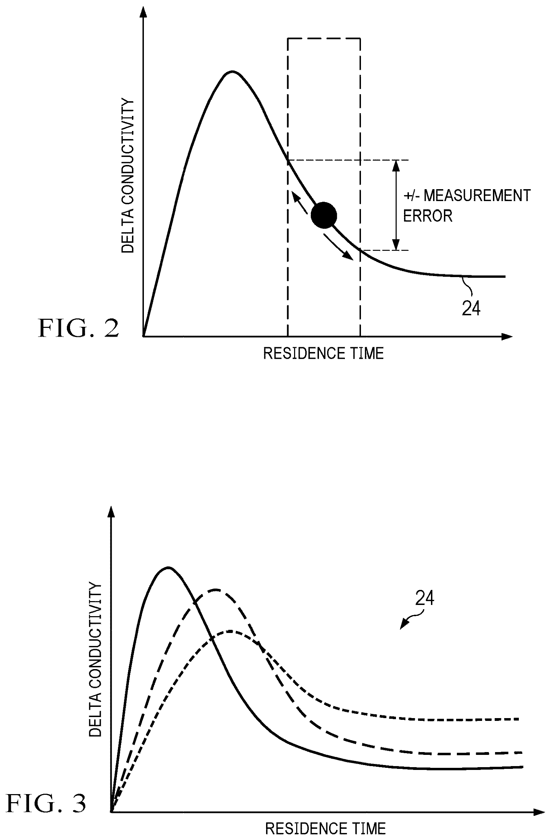

As demonstrated with particular regard to **FIGURE 2,** the delta conductivity measurement may vary with residence time of the sample fluid 16 within the first portion of the sample fluid passageway 12, commonly resulting in an oxidation curve 24 generally of a shape illustrated. This may be, for example without limitation, different species within the sample fluid 16 may oxidize into different chemical compounds over prolonged exposure, generally ultimately oxidizing into $CO_2$ if exposed for a sufficiently long period of time. Thus, as exposure/residence time varies, so will (generally) delta conductivity measurements. However, as further demonstrated with particular regard to **FIGURE 3,** various size and/or shape oxidation curves 24 may be generated, as such measurements are affected by various unpredictable and/or random factors, such as but not necessarily limited to, composition of the sample fluid 16, flow rate, gas bubble interference, UV dosage, temperature of the sample fluid 16, combinations thereof, or the like. Errors may persist even at complete oxidation, such as if all the reactant did not exclusively generate $CO_2$ and/or if the reactant was a conductive organic substance. The foregoing is not a comprehensive listing of error possibilities.

Longer residence times tend to result in more accurate measurements for at least certain interfering species. However, other interfering species can still cause errors further out on the oxidation curve. Regardless, longer residence times are undesirable as they interfere with production speed. Lower residence time may result in inaccurately high or low measurements, especially as the measurements shift due to variability in other factors (e.g., flow rate, gas bubble interference, UV dosage, temperature of fluid), examples of which are designated in figure 2, without limitation.

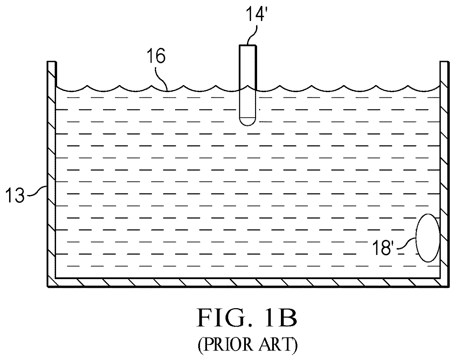

Traditionally, an oxidation curve for a particular sample could only be developed by batch processing and exposing a sample batch to some oxidizing condition over time (e.g., adding reagents, combustion with catalyst, UV exposure time, recycling oxidation loop, electrochemical oxidation, combinations thereof, or the like). For example, as illustrated with particular regard to **FIGURE 1B,** the sample fluid 16 may be provided within a container 13 with a conductivity sensor 18' and a UV light source 14' proximate or within the container, and multiple data points are gathered as the sample is increasingly exposed to the UV light. Such approaches necessarily take additional time to gather the desired data. Traditionally, active (e.g., continuous) flow sensor device operate flow at, or substantially at (e.g., within 2% of), a given flow rate, resulting in a steady oxidation exposure/residence time and a resulting single data point measurement.

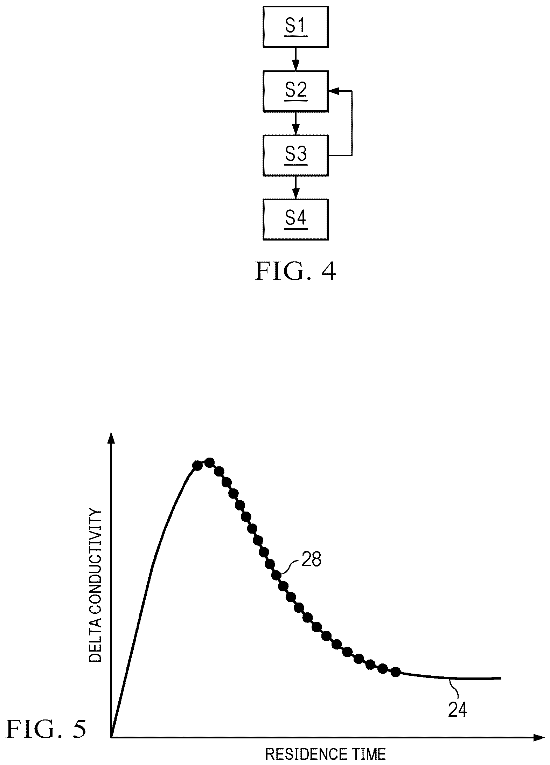

**FIGURE 4** illustrates a method for generating multiple data points for a flow of the sample fluid 16 at the TOC device 10, such as to generate an oxidation curve, such as while maintaining active (e.g., continuous) flow at the TOC device 10.

In exemplary embodiments, the following steps are performed: S1 - initiate sensor readings (ongoing), S2 - cause a high flow rate for a first period of time, S3 - cause a low flow rate for a second period of time, and S4 generate a TOC analysis from sensor readings. Steps S2 and S3 may be repeated, such as in a cyclical fashion.

The flow of the sample fluid 16 through the sample fluid passageway 12, or at least the first portion thereof, may be provided at different flow rates over time. In exemplary embodiments, without limitation, the sample fluid 16 is provided at a relatively high flow rate for a first period of time. For example, without limitation, the sample fluid 16 may be provided at a flow rate between 10 and 100 ml/min for a time period between 1 and 125 seconds. The flow may subsequently be switched to a relatively low flow rate for a second period of time. For example, without limitation, the sample fluid 16 may be provided at a rate of between 1 and 30 ml/min for a time period between 1 and 125 seconds. The first and second periods of time may be the same or different. The flow rates and times shown and/or described herein are exemplary and not intended to be limiting. In exemplary embodiments, without limitation, the flow rates and times may be adjusted based on various characteristics of the TOC device 10 (e.g., sample fluid passageway 12 size and/or shape) such as to provide a variety of residence times of the sample fluid 16 within the sample fluid passageway 12 so as to generate the desired data. The relatively low flow rate may provide the sample fluid 16 with a first residence time within the sample fluid passageway 12, while the relatively high flow rate may provide the sample fluid 16 with a second residence time within the sample fluid passageway 12, which is shorter than the first residence time. Alternatively, or additionally, a volume of the sample fluid 16 at the high flow rate may be provided through the TOC device 10 which at least equals, or is up to 5 times, preferably about three times, a volume of the sample fluid passageway 12 of the TOC device 10. In exemplary embodiments, without limitation, the high and low flow rates will continue in a cyclical, preferably continuously cyclical, fashion by a bimodal, square-wave pattern, combinations thereof, or the like.

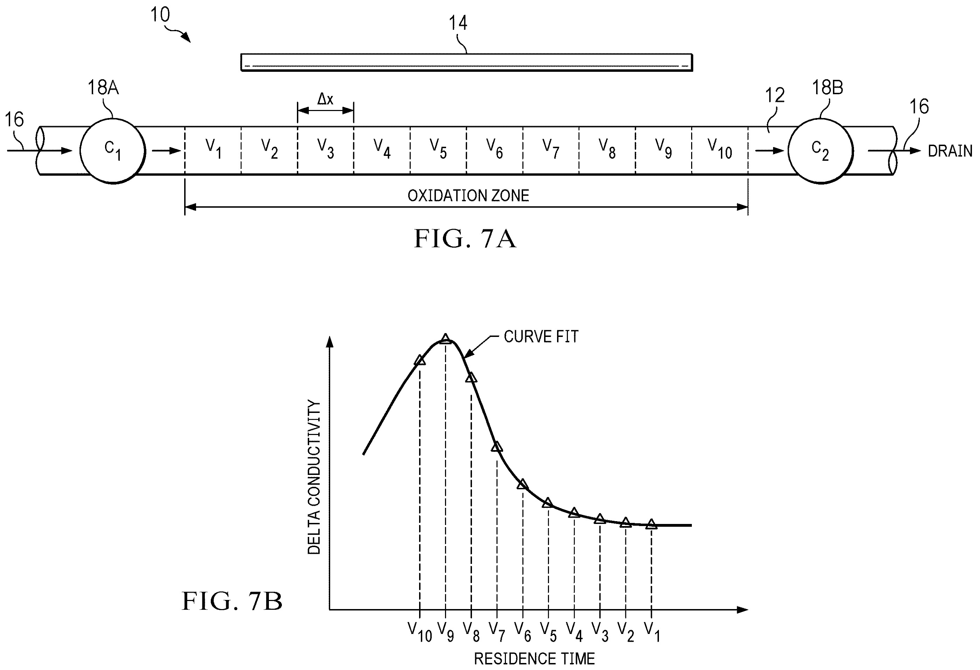

As illustrated with particular regard to **FIGURE 7A** and **FIGURE 7B,** and further discussed herein, at one or more times while the different flow rates are underway, measurements may be taken by the sensors 18. With particular regard to **FIGURE 7A,** as the sample fluid 16 passes from an intake or entrance through the sample fluid passageway 12, at least a portion of which is exposed to the light source 14 (hereinafter sometimes also called the "oxidation zone"), the sample fluid 16 may be exposed to various levels of oxidation over time before exiting the sample fluid passageway 12 by way of a drain or exit. Sensor 18 data may be taken at continuous intervals while the TOC device 10 is operational (e.g., while the flow rate is changed), only before and/or after flow changes, or the like. "V" (i.e., $V_1$, $V_2$, $V_3$, etc.) may represent discrete volumes of the sample fluid 16, each of which may have a different value when measured by the sensor(s) 18, where "x" may represent the size of the volume of the sample fluid 16 and/or the sample fluid passageway 12 containing the sample fluid 16 under consideration. After switching from the high flow to the low flow, the first portion of the sample fluid 16 within the sample fluid passageway 12 that is measured by the conductivity sensor 18B will yield a $\Delta C$ value corresponding to "V10", by way of example. This $\Delta C$ value will also correspond to a unique residence time reflective of the high flow. Subsequently each additional portion of fluid as indicated by "V9" , "V8", "V7", etc. will correspond to increasing unique residence times reflective of a combination of the high and low flows along with the corresponding $\Delta C$ values. As $\Delta x$ decreases to the size of a differential element, the conductivity sensor 18B is able to measure an increasingly larger number of differential sample fluid volumes (dV) with each differential sample fluid volume corresponding to a unique ordered pair comprised of a residence time and a corresponding $\Delta C$ value. The number of ordered pairs generated in the TOC device 10 represents a unique oxidation curve for the sample fluid 16 within the sample fluid passageway 12. The number of discrete data points representing the oxidation curve in further analysis may be determined by the data log settings located in the controller 20. With particular regard to **FIGURE 7B,** the multiple data points from the sensors 18 (e.g., $V_1$, $V_2$, $V_3$, etc.) may be curve fit, such that the multiple data points could be graphically represented in the form of $\Delta C$ values ($C_2 - C_1$) on the Y-axis against residence time of the sample fluid 16 on the x-axis.

In exemplary embodiments, without limitation, at least one measurement is taken while at least the first portion of the sample fluid passageway 12 is filled with only the relatively high flow rate sample fluid 16, at least one other measurement is taken while at least the first portion of the sample fluid passageway 12 is filled with a combination of high and low flow rate sample fluid 16 (e.g., the high flow rate sample fluid 16 is being displaced by the low flow rate sample fluid 16), and at least one other measurement is taken while at least the first portion of the sample fluid passageway 12 is filled with only the relatively low flow rate sample fluid 16. However, a large variety of measurements may be taken at various times, such as to reflect various residence times of the sample fluid 16.

Alternatively, or additionally, the low flow rate may be first introduced with the high flow rate after. In this fashion, the TOC device 10 may operate with a cyclic and/or bimodal flow, which is an exemplary embodiment. Alternatively, or additionally, a larger number of different flow rates may be provided (e.g., high, medium, and low, four different flow rates, etc.) , such as in a cyclical, preferably continuously cyclical, fashion. The flow rates may be provided as step-changes, gradual changes, sinusoidal changes, saw tooth changes, combinations thereof, or the like. Regardless, alternating and/or changing between the different flow rates may be repeated, in a same or different order.

In yet other exemplary embodiments, without limitation, a plurality of conductivity sensors 18 may be posited along the sample fluid passageway 12 which may have a constant flow rate of the sample fluid 16 over time. This may provide a plurality of conductivity measurements reflecting various residence and/or exposure times of the sample fluid 16 to the light source 14.

Regardless of how the multiple data points are generated, multiple data points may be taken of portion(s) of the sample fluid 16 having different residence times in the sample fluid passageway 12 (and therefore different UV exposure/oxidation levels from the light source 14). The multiple data points may be collectively formed into a regression curve shape and/or a curve reflective of a complete oxidation curve, or part of an oxidation curve, such as illustrated with particular regard to **FIGURE 5** at item 28. In this way, the multiple data points may be reflective of the oxidation curve of the sample fluid 16, as generated by the TOC device 10.

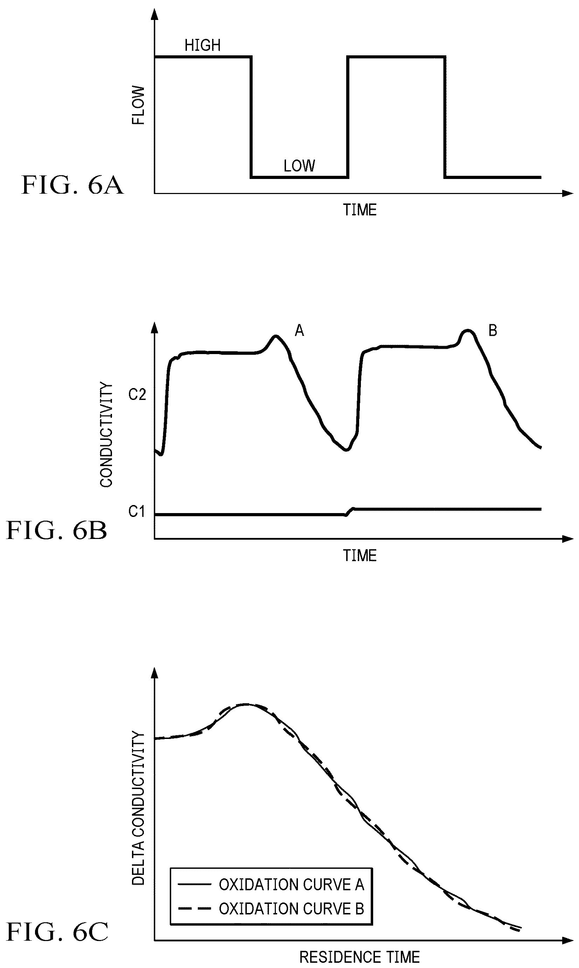

Examples of such data measurements and TOC device 10 operations are illustrated with regard to **FIGURE 6A** through **FIGURE 6C** by way of non-limiting example. As illustrated generally at **FIGURE 6A**, the TOC device 10 may be configured to automatically (e.g., in a pre-programmed or otherwise controlled fashion) switch between two (or more) different flow rates (e.g., high, low) over time, such as to provide a multimodal (e.g., bimodal) cycle of different flow rates. As illustrated generally at **FIGURE 6B** this may result in a continuous oxidation curve as the oxidizing species within the sample fluid 16 oxidizes over time based on exposure and/or residence time, such changes being represented by discrete conductivity measurements by the sensors 18. Those measurements may be transformed into delta conductivity measurements, such as at the controller 20, as illustrated generally at **FIGURE 6C.** Actual measurements/ multiple data points may represent discrete gathered conductivity measurements over time. A curve may be fit to those discrete multiple data points, or such multiple data points may be fit to a curve. Examples of such data measurements are illustrated with regard to **FIGURE 7A** and/or **FIGURE 7B,** without limitation.

These steps (e.g., switching between relatively high and low flow rates) may be repeated to generate additional curves and/or gather additional data points over a continuous flow of the sample fluid 16. For instance, a continuous, active flow of the sample fluid 16 may be passed through the TOC device 10 at the different flow rates to generate the multiple data points for the (complete or partial) oxidation curve. In this regard, an assumption may be made that the sample fluid 16 is the same, or substantially the same (e.g., in composition, temperature, etc.), over the time measured.

Other techniques for altering residence/exposure times may be utilized. For example, without limitation, the sample fluid 16 may be recycled through the TOC device 10 (at a same or different flow rate) to differentially expose the sample fluid 16 to ultraviolet light from the light source 14. As another example, without limitation, an electrochemical oxidation device may be utilized at a constant flow. Conductivity measurements may be taken by sensor 18 located at, or proximate, the inlet and outlet of the electrochemical oxidation device. By varying operating parameters of the electrochemical oxidation device (e.g., current, voltage, etc.) differing oxidation conditions are generated.

In exemplary embodiments, without limitation, the different flow rates (e.g., switching between high and low flow rates) may be selectively initiated, such as automatically at certain times, periodically, manually, in response to certain conditions, combinations thereof, or the like. For example, without limitation, the TOC device 10 may be configured to automatically initiate the different flows and subsequent operations (e.g., gather data, process results) upon start-up of the larger system, regularly during operation, randomly, combinations thereof, or the like. As another example, without limitation, the TOC device 10 may be configured to normally operate at a baseline flow (e.g., low flow rate, high flow rate, different flow rate above the low flow rate but below the high flow rate) until the TOC device 10 or another component (e.g., sensor) of the larger system otherwise detects an excursion (e.g., by TOC measurement, by chemical analysis, temperature change, pH level change, other sensor data, etc.) and subsequently activates the different flow rates and related operations unless/until the excursion event is no longer detected.

The differential oxidation exposure approach herein described may reduce sensitivity to various, otherwise error inducing factors including, but not necessarily limited to, flow rate variability, lamp power output, temperature variability, gas bubbles, and background conductivity, combinations thereof, or the like. This approach may provide superior performance on under- or over-reporting at least the following: KHP, SDBS, IPA, MeOH, chloroform, and Urea, by way of non-limiting example. This approach may provide superior accuracy, repeatability, ease of calibrations, and/or improved response time, by way of non-limiting example. This approach may facilitate the generation of full or partial oxidation curves and/or multiple data points for iterative model learning and improvement, identification, and/or classification, by way of non-limiting example.

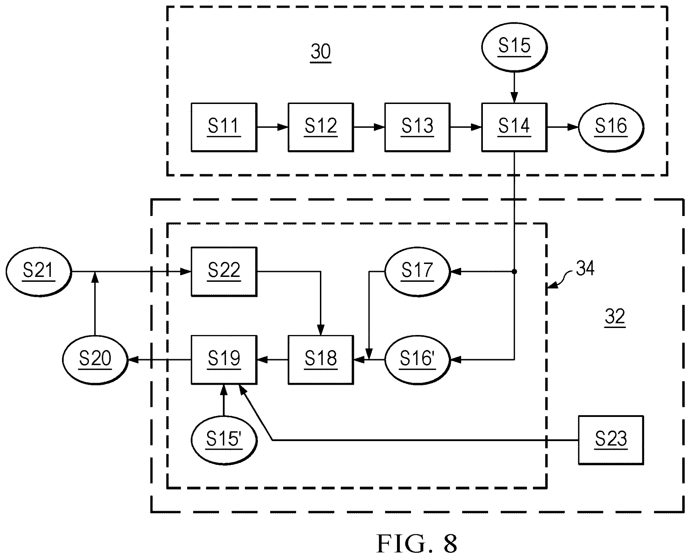

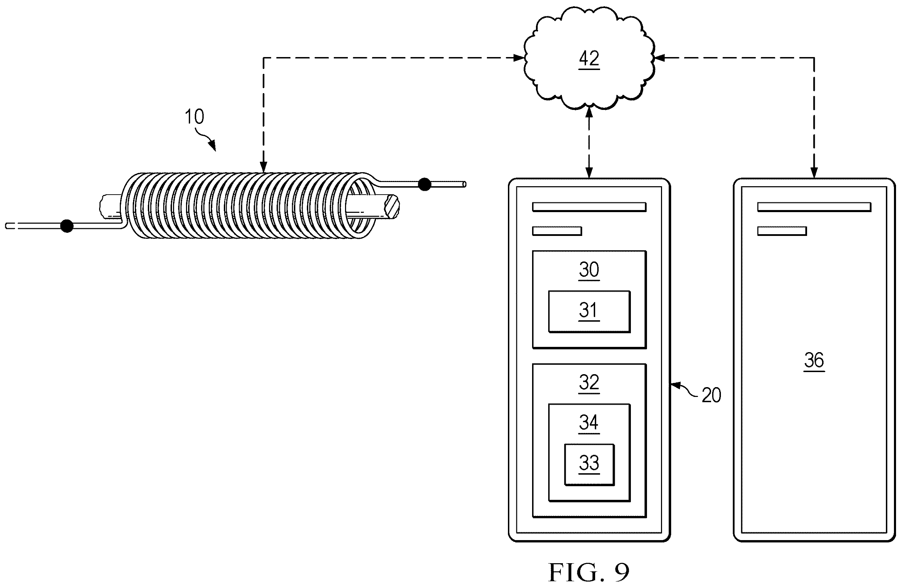

As illustrated with particular regard to **FIGURE 8** and **FIGURE 9,** the multiple data points gathered may be analyzed, such as at the controller 20.

Some or all of the controller 20 may be local to the TOC device 10 or remote therefrom and in communication with the TOC device 10 by way of one or more networks 42.

The analysis performed by the controller 20 may be performed by way of a stochastic analysis module 32, which may include an artificial intelligence module 34, and a deterministic analysis module 30 in exemplary embodiments. The deterministic analysis and stochastic analysis modules 30, 32 may be part of the controller 20 and/or TOC device 10, or located separately therefrom and in communication with the controller 20 and/or TOC device 10 by way of one or more networks 42. While used together, in exemplary embodiments, each of the deterministic analysis and stochastic analysis modules 30, 32 may be separately utilized in other exemplary embodiments. Each of the deterministic analysis and stochastic analysis modules 30, 32 may be configured to, in an at least partially automated fashion based on executed software instructions, carry out various steps as shown and/or described herein. The disclosed steps may be performed in different orders, may be omitted, repeated, or the like. While sometimes shown and/or described as separate modules, any number of modules may be utilized, including combining the deterministic analysis and stochastic analysis modules 30, 32 into a common module, or further breaking apart the deterministic analysis and stochastic analysis modules 30, 32 into submodules.

The controller 20 may comprise, or may be in electronic communication with, an expert knowledge system 36 as further described herein.

In exemplary embodiments, without limitation, raw data is acquired at step S11, the data is reduced at step S12, a deterministic model 31, such as of the deterministic analysis module 30, is applied at step S13, and inference with the deterministic model 31 is determined at step S14 to output an initial TOC measurement at step S16, such as in response to a query at step S15 regarding what is the TOC value. Some or all of these steps (S11 through S16) may be performed as part of execution of the deterministic analysis module 30, though such is not required.

In exemplary embodiments, without limitation, following, and optionally in response to step S15, the initial TOC measurement may be provided to the artificial intelligence module 34 of the stochastic analysis module 32 at step S16'. Additionally, an N dimensional tensor may be provided from the deterministic model 31, such as where N >= 0, at step S17, along with the initial TOC measurement of step S16'. A stochastic error model 33 may be applied at step S18. Inference with the stochastic error model 33, application of the expert knowledge system 36, such as by query at step S23, tensor, and initial TOC measurement may be applied at step S19, such as in response to a query regarding what is the TOC error at step S15', to output the error compensated TOC measurement at step S20. The error compensated TOC measurement and/or optionally any user input provided at step S21, may be fed back to the stochastic error model 33, such as by way of a self-learning feedback loop at step S22. Step S23 may optionally occur outside of the artificial intelligence module 34 but as part of the stochastic analysis module 32.

Such analysis may be performed while some or all of the steps of figure 4 are underway or thereafter, by way of non-limiting example. While, at times, a discussion may be made of analyzing multiple data points gathered using the TOC device 10 and/or method of figure 4, the data analysis methods shown and/or described herein may be utilized with multiple data points gathered by other types and/or kinds of TOC sensor devices and/or methods (e.g., batch processing), such as but not necessarily limited to as shown and/or described in US Pat. No. 5,275,957 and/or US Pat. No. 8,618,820, the disclosures of which are hereby incorporated by reference.

As part of executing the deterministic analysis module 30, data, such as raw data, may be acquired at step S11, such as from the sensors 18. The multiple data points may include conductivity measurements from the sensors 18 over time or other sensor data. For example, without limitation, the multiple data points may include conductivity measurements with associated recorded times. The time of the measurement may be recorded by the sensors 18, at the controller 20, by a separate timing device, combinations thereof, or the like.

The data may be reduced at step S12, such as at the controller 20 as part of the deterministic analysis module 30, such as to generate multiple data points. For example, without limitation, such data reduction may include determining a difference in conductivity for sensor 18 measurements, taking an absolute value of the difference, removing noise, and/or eliminating erroneous measurements (e.g., those above/below certain predetermined thresholds, outside a certain number of standard deviations, combinations thereof, or the like), by way of non-limiting example.

The multiple data points may be processed assuming the absence of unpredictability and randomness at step S13, such as part of the deterministic model 31 and/or extrapolation technique. Optionally, an initial TOC measurement may be provided following such processing at step S16. In exemplary embodiments, the deterministic analysis module 30 may use known compensation and/or conversion techniques to convert the delta conductivity measurement to the initial TOC measurement and/or a ppbC measurement, or other measurement. The deterministic model 31 at step S13 may utilize, for example without limitation, certain loss function minimization methods, peak detection techniques, signal integration techniques, combinations thereof, or the like as part of the data analysis. Alternatively, or additionally, the multiple data points may be fit to a curve and/or a curve may be fit to the data, such as using one or more known curve fitting techniques. In exemplary embodiments, without limitation, such curve fitting techniques include a multi-parameter, weighted, regression analysis to essentially fit a curve to the multiple data points, where each parameter represents a quantified state of the oxidation curve. However, other techniques may alternatively or additionally be used such as lookup tables, other curve fitting models, curve identification models, curve extrapolation models, combinations thereof, or the like. Alternatively, or additionally, a regression analysis, extrapolation analysis, or the like may be performed.

Determination and/or provision (e.g., display to an end user) of the initial TOC measurement may occur automatically, and/or in response to a user and/or automated (e.g., by controller 20, with or without manual user input) inquiry at step S15.

As indicated at step S14, in exemplary embodiments, without limitation, a stochastic analysis module 32 may be utilized to help correct for unpredictability and/or randomness caused by the presence/non-presence of various interfering species, temperature changes, UV dosage changes, combinations thereof, or the like, by way of example. At step S16' the initial TOC measurement from the deterministic analysis module 30 may be provided to the stochastic analysis module 32. The stochastic analysis module 32 may allow for learning applications, such as with supervised user input stored at an expert knowledge system 36, though such is not required. The expert knowledge system 36 may comprise, or access, one or more databases which form part of, and/or are accessible by, the stochastic analysis module 32 and/or the deterministic analysis module 30.

The stochastic analysis module 32 may include the artificial intelligence module 34. The multiple data points and/or initial TOC measurement may be processed through the stochastic analysis module 32, including through the artificial intelligence module 34, such as to ultimately arrive at an error compensated TOC measurement (hereinafter also "TOC output") at step S20. As described herein, the error compensated TOC measurement may be a measurement which compensates for multiple or all error parameters.

In exemplary embodiments, without limitation, the artificial intelligence module 34 may be developed by providing a series of training data sets to one or more stochastic error models 33 at step S18, which may comprise one or more neural networks. The training data sets may be manually classified and may represent certain controlled conditions, such as samples with known compositions, at known temperatures, at known flow rates, combinations thereof, or the like. Alternatively, or additionally, the same or different training data sets may be provided to the expert knowledge system 36. The artificial intelligence module 34 may be configured to develop, comprise, and/or utilize a multi-dimensional error analysis hypersurface, such as, but not necessarily limited to, a three-dimensional error analysis mesh, representing the parameters of the deterministic model 31 and error. The artificial intelligence module 34 may utilize an N dimension tensor, where N >= 0 at step(s) S17, S19, and/or an expert knowledge system 36 at step S19 (optional) to determine an error, such as absolute error, which is applied to the initial TOC measurement at steps S18 and S19 to arrive at the error compensated TOC measurement at step S20. Determination and/or provision (e.g., display to an end user) of the error compensated TOC measurement may occur automatically, and/or in response to a user and/or automated inquiry at step S15'.

The stochastic analysis module 32 may be configured to apply at least certain of the parameters of the deterministic model 31 of step S13 to the multi-dimensional error analysis hypersurface to derive an error for the initial TOC measurement at step(s) S18 and/or S19. The stochastic analysis module 32 may adjust the initial TOC measurement by the derived error to determine the error compensated TOC measurement as provided at step S20. The error compensated TOC measurement may be further compensated as required and provided in and/or converted to ppbC or another measurement. The error compensated TOC measurement may be output and/or converted to various formats (e.g., qualitative or quantitative) using known techniques before final output, though such is not necessarily required.

The expert knowledge system 36 may comprise, or access, one or more databases with one or more rules such as, but not limited to, parameters of the error analysis correlating with certain compounds of interfering species, user specific rules, application specific rules, acceptance or rejection of the oxidation curve data for further artificial intelligence analysis, acceptance or rejection of the regression parameters for further artificial intelligence analysis, rules regarding whether and when to use the artificial intelligence module 34 or another algorithm, using limits to control adjustment to the multi-dimensional error analysis hypersurface (e.g., 3D error analysis mesh) during application of a self-learning stochastic feedback loop at step(s) S22 and S18, such as with user input as optionally provided at step S21, storing rules for intelligently monitoring and/or controlling a user's water system based on analytic results generated from the artificial intelligence module 34 or another algorithm, combinations thereof, or the like, though any type or kind of rules may be provided. The expert knowledge system 36 can be queried by the artificial intelligence module 34 and/or the deterministic analysis module 30 at any point during the disclosed method, preferably at or between one or more of steps S18-S20.

The stochastic analysis module 32 may utilize the self-learning feedback loop at step S22. In exemplary embodiments, without limitation, the self-learning feedback loop may permit a user to manually adjust error and/or TOC output or otherwise provide other input or rules (e.g., user specific training data sets and/or user specific rules) which are adopted into the artificial intelligence module 34 and/or the expert knowledge system 36, accordingly, such as to provide user specific error compensated measurements. Alternatively, or additionally, the self-learning feedback loop may permit control over various components of the TOC device 10 and/or larger system components (e.g., end user system or components thereof to which the TOC device 10 is connected) based on the findings of the artificial intelligence module 34 (e.g., error compensated TOC measurement or other findings). The information from the self-learning feedback loop may be provided at step S22 to the stochastic error model 33, such as for additional runs of the stochastic analysis module 32 to improve the stochastic analysis at step S19.

While, at times, a discussion may be made of analyzing the multiple data points gathered using the TOC device 10 and/or method of figure 4, the data analysis methods shown and/or described herein may be utilized with other types of sensors, devices, and/or methods (e.g., batch processing, sensors which separate $CO_2$, combinations thereof, of the like). In the case of sensors, devices, and/or methods which separate $CO_2$, the artificial intelligence analysis module 34 may be used to compensate for variability in transfer rate of $CO_2$, environmental conditions, combinations thereof, or the like, by way of example.

In exemplary embodiments, without limitation, the analysis (e.g., generation of oxidation curves, TOC measurements, ppbC measurements, combinations thereof, or the like) are made in real-time. As used in herein, the term real-time may account for normal delays in data transmission and processing, but may exclude deliberate delays of over 1 minute, by way of non-limiting example.

The data analysis methods shown and/or described herein may be performed at the controller 20 or elsewhere (e.g., remote computers, user devices, servers, processors, combinations thereof, or the like). The controller 20 may constitute a single device in a single location, or a single, operational unit which is physically located at disparate locations (e.g., software code and/or other data stored at non-transitory electronic storage devices at more than one location and processed by processors at more than one location).

The results of the analysis, such as in the form of an oxidation curve, TOC measurement(s) (in ppbC or otherwise), multiple data points, insights (e.g., classification results), combinations thereof, or the like, may be visualized at an electronic display 21 local to the TOC device 10, remote therefrom (e.g., as part of a remote user device 23, such as but not necessarily limited to, computer, smartphone, tablet, etc.), combinations thereof, or the like. The results of the analysis may be displayed in real-time, historically, in summary form, combinations thereof, or the like. The results of the analysis may be displayed in quantitative and/or qualitative form, and/or in various formats (numerical measurement, spreadsheet, csv file, graphical plot, etc.). Connection between the controller 20 and the display 21, user device 23, and/or TOC device 10 or components thereof (e.g., sensors 18 and/or flow control device(s) 22) may be made by way of wired and/or wireless connection, such as by way of one or more networks 42, which may comprise the internet, intranet, cellular network, combinations thereof, or the like. Data for and/or from the analysis and/or results of the analysis may be stored at one or more local or remote databases, servers, combinations thereof, or the like. Data for and/or from the analysis and/or results of the analysis may be accessible via website, portal, application, combinations thereof, or the like.

The analysis may be made of various experimental parameters, such as with various temperatures, UV/light dosage, various sample fluids 16, various interfering species, combinations thereof, or the like. Such parameters may be obtained under controlled conditions, such as for use as training data for the artificial intelligence module 34 (e.g., for classification purposes), by way of non-limiting example.

While application to measuring conductivity, such as by way of the sensors 18, is sometimes shown and/or described, other types and kinds of sensors 18 and data measurements may be taken, such as but not limited to: pH, ion selective, NDIR, and other measurement of oxidation products. Data measurements may be derived by converting data from the sensors 18 into various formats, such as converting from uS/cm to ppbC. Additionally known mathematical functions and/or algorithms may be applied to the sensor 18 outputs or applied to data derived from a combination of the sensor 18 outputs during the process of deriving a TOC measurement.

The TOC device 10, such as the controller 20, may be configured to provide alerts, such as electronic notification, upon analysis results (e.g., TOC measurements) meeting certain predetermined rules (e.g., requirements, limits, changes, indications of particular compounds present, combinations thereof, or the like, which may be user defined. The controller 20 may be configured to transmit such alerts to user devices, such as those affiliated with administration of the larger system, and/or to other controllers, such as those affiliated with the larger system, such as to make operational command decisions based on the same in a manual or automated fashion.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention. Many of those variations and modifications will provide the same result and fall within the spirit of the claimed invention.

Certain operations described herein may be performed by one or more electronic devices. Each electronic device may comprise one or more processors, electronic storage devices, executable software instructions, combinations thereof, and the like configured to perform the operations described herein. The electronic devices may be general purpose computers or specialized computing devices. The electronic devices may comprise personal computers, smartphones, tablets, databases, servers, or the like. The electronic connections and transmissions described herein may be accomplished by one or more wired or wireless connectivity components (e.g., routers, modems, ethernet cables, fiber optic cable, telephone cables, signal repeaters, and the like) and/or networks (e.g., internets, intranets, cellular networks, the world wide web, local area networks, and the like). The computerized hardware, software, components, systems, steps, methods, and/or processes described herein may serve to improve the speed of the computerized hardware, software, systems, steps, methods, and/or processes described herein. The electronic devices, including but not necessarily limited to the electronic storage devices, databases, controllers, or the like, may comprise and/or be configured to hold, solely non-transitory signals.

**Claims**

1. A system for providing artificial intelligence analysis of total organic carbon ("TOC") sensor data, said system comprising:

   a TOC measurement device (10) comprising:

      a fluid containing portion (12) for holding a sample fluid (16);
      one or more sensors (18) at the fluid containing portion (12); and
      an oxidation device (14) at the fluid containing portion (12); and

   a controller (20) comprising one or more non-transitory electronic storage devices comprising software instructions, which when executed, configure one or more processors to:

      operate the oxidation device (14) to cause the sample fluid (16) at the fluid containing portion (12) to experience differing levels of exposure to the oxidation device over a time period;
      receive a plurality of measurements from the one or more sensors (18) over the time period, each taken at a different time such that each of the measurements reflect a different exposure level of the sample fluid from the oxidation device (14);
      analyze the plurality of measurements, including by applying a deterministic model, to determine an initial TOC measurement for the sample fluid (16); and
      apply an artificial intelligence module to the initial TOC measurement to determine an error compensated TOC measurement.

2. The system of claim 1, wherein:
   the error compensated TOC measurement is provided in, or converted to, ppbC or another format.

3. The system of claim 1 or 2, wherein:
   the deterministic model comprises a multi-parameter, weighted, regression analysis which fits the plurality of measurements to a curve, wherein preferably the artificial intelligence module utilizes a multi-dimension tensor to store regression analysis parameters from the deterministic model.

4. The system of one of the preceding claims, wherein:
   the artificial intelligence module is developed with manually classified training data sets with controlled specification, including sample fluid composition which develop a multi-dimensional error analysis hypersurface representing the parameters and error, wherein preferably the artificial intelligence module comprises a stochastic error model which applies the multi-dimensional error analysis hypersurface to the parameters of the deterministic model and adjusts the initial TOC measurement to the error compensated TOC measurement based on the resulting error value.

5. The system of one of the preceding claims, further comprising:
   an expert knowledge system comprising one or more databases comprising rules, wherein the artificial intelligence module or the deterministic model is configured to query the expert knowledge system prior to determining the error compensated TOC measurement, said rules comprising at least one rule for classification of at least one chemical type.

6. The system of one of the preceding claims, wherein:

      said stochastic error model comprising a self-learning stochastic feedback loop; and
      the controller comprises additional software instructions stored at the one or more non-transitory electronic storage devices, which when executed, configures the one or more processors to:

receive data indicating verification or denial of the error compensated TOC measurement; and
update the stochastic error model in accordance with the data indicating verification or denial of the error compensated TOC measurement as part of the self-learning stochastic feedback loop.

7. The system of one of the preceding claims wherein:
the TOC measurement device (10) comprises an active flow device.

8. The system of one of the preceding claims wherein:

the fluid containing portion (12) comprises a flow channel;
the oxidation device (14) comprises an ultraviolet ("UV") lamp;
the TOC measurement device (10) comprises a flow control device (22) configured to control a flow rate of the sample fluid through the flow channel; and
the controller (20) comprises additional software instructions stored at the one or more non-transitory electronic storage devices, which when executed, configures the one or more processors to: alter the flow rate of the sample fluid through the flow channel in a cyclical fashion while the plurality of measurements is taken and the oxidation device (14) is active, wherein preferably the flow channel is shaped into a coil about the UV lamp.

9. The system of one of the preceding claims wherein:

the TOC measurement device (10) comprises a batch processing device;
the one or more sensors (18) comprise conductivity sensors;
the fluid containing portion (12) comprises a fluid chamber configured to statically hold the sample fluid for a period of time;
the oxidation device (14) comprises at least one of: an ultraviolent ("UV") lamp, a reagent dispenser, a heating element, a catalyst device, and electrochemical device; and
the controller comprises additional software instructions stored at the one or more non-transitory electronic storage devices, which when executed, configures the one or more processors to: take the plurality of measurements at different times.

10. The system of one of the preceding claims wherein:

the controller (20) is remote from the TOC measurement device (10) and in electronic communication with at least one component of an end-user system to which the TOC measurement device (10) is fluidly connected; and
the controller (20) comprises additional software instructions stored at the one or more non-transitory electronic storage devices, which when executed, configures the one or more processors to: command operation of the at least one component of the end-user system.

11. A method for providing artificial intelligence analysis of total organic carbon ("TOC") sensor data, said method comprising:

operating a TOC measurement device (10) to cause differing exposure of a sample fluid at a fluid containing portion (12) of the TOC measurement device (10) from an oxidation device (14) of the TOC measurement device over time;
receiving, at a controller (20), a plurality of measurements from one or more sensors (18) of the TOC measurement device (10) at the fluid containing portion (12) of the TOC measurement device (10) over time such that each of the plurality of measurements reflect a different exposure level of the sample fluid (16) to the oxidation device (14);
analyzing, by way of the controller (20), the plurality of measurements, including by applying a deterministic model to the plurality of measurements, to determine an initial TOC measurement for the sample fluid, and by applying an artificial intelligence module to the initial TOC measurement to determine an error compensated TOC measurement.

12. The method of claim 11, further comprising:

developing the artificial intelligence module with manually classified training data sets with controlled specification, including sample fluid composition which develop a multi-dimensional error analysis hypersurface representing multiple parameters and error, wherein the deterministic model comprises the multiple parameters as part of a weighted, regression analysis which fits the plurality of measurements to a curve; and

applying, by way of the artificial intelligence module and the controller, the multi-dimensional error analysis hypersurface to the parameters of the deterministic model, and adjusting, by way of the controller (20), the initial TOC measurement to the error compensated TOC measurement based on the resulting error value, wherein preferably the error compensated TOC measurement is provided in, or converted into, a ppbC measurement.

13. The method of claim 11 or 12, further comprising:

querying, by way of the controller (20), an expert knowledge system prior to determining the error compensated TOC measurement, said expert knowledge system comprising one or more databases comprising rules, including at least one rule for classifying at least one chemical type, wherein preferably the method further comprises: receiving, by way of the controller (20), data indicating verification or denial of the error compensated TOC measurement; and

updating, by way of the controller (20), the multi-dimensional error analysis hypersurface of the stochastic error model in accordance with the data indicating verification or denial of the error compensated TOC measurement as part of the self-learning stochastic feedback loop.

14. The method of one of the claims 11 to 13, wherein:

the TOC measurement device (10) comprises an active flow device;
the fluid containing portion (12) comprises a sample fluid passageway; and
the step of operating the TOC measurement device (10) to cause differing exposure of the sample fluid (16) at the fluid containing portion of the TOC measurement device (10) to the oxidation device (14) of the TOC measurement device (10) over time comprises causing a cyclic, active flow rate at the sample fluid passageway (12) over time.

15. A system for providing artificial intelligence analysis of total organic carbon ("TOC") sensor data, said system comprising:

a TOC measurement device (10) comprising:

a fluid sample passageway (12) configured to hold a sample fluid (16);
one or more sensors (18) located at the fluid containing portion (12);
an oxidation device (14) at the fluid containing portion (12); and
a flow control device (22) located at the fluid containing portion (12); and

a controller (20) comprising one or more non-transitory electronic storage devices comprising software instructions, which when executed, configure one or more processors to:

operate the flow control device (22) to cause a cyclic, non-zero, active flow rate of the sample fluid (16) through the fluid sample passageway (12) over time;
receive a plurality of measurements from the one or more sensors (18), where each of the plurality of measurements are taken at a different time such that each of the measurements reflect a different oxidation level of the sample fluid;
fit the plurality of measurements to a curve to generate a regression curve for the sample fluid;
apply a deterministic model to the plurality of measurements to determine an initial TOC measurement for the sample fluid (16); and
apply an artificial intelligence module to the initial TOC measurement to determine an error compensated TOC measurement.

FIG. 1A

**FIG. 1B**
(PRIOR ART)

FIG. 2

DELTA CONDUCTIVITY

RESIDENCE TIME

+/- MEASUREMENT ERROR

24

FIG. 3

DELTA CONDUCTIVITY

RESIDENCE TIME

24

FIG. 4

FIG. 5

FIG. 6A

FIG. 6B

FIG. 6C

OXIDATION CURVE A
OXIDATION CURVE B

FIG. 7A

FIG. 7B

EP 4 722 716 A1

FIG. 8

FIG. 9

Europäisches
Patentamt

European
Patent Office

Office européen
des brevets

## EUROPEAN SEARCH REPORT

**Application Number**

EP 25 20 5545

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 102 541 059 B1 (BIN TECH KOREA CO LTD [KR]) 13 June 2023 (2023-06-13) | 1-9, 11-13 | INV. G01N33/18 G06N20/00 |
| Y | * paragraph [0001] * <br> * paragraph [0012] * <br> * paragraph [0019] * <br> * paragraph [0038] * <br> * paragraph [0048] - paragraph [0049] * <br> * paragraph [0052] * | 10,15 | |
| Y | CN 201 392 338 Y (UNIV BEIJING TECH & BUSINESS) 27 January 2010 (2010-01-27) | 10,15 | |
| A | * the whole document * | 7,8 | |
| A | CN 105 911 003 A (UNIV CHONGQING) 31 August 2016 (2016-08-31) <br> * the whole document * | 1-15 | |
| A | US 6 451 613 B1 (BLADES FREDERICK K [US] ET AL) 17 September 2002 (2002-09-17) <br> * the whole document * | 1-15 | |

**TECHNICAL FIELDS SEARCHED (IPC)**

G01N
G06E
G06N

The present search report has been drawn up for all claims

| Place of search | Date of completion of the search | Examiner |
|---|---|---|
| Munich | 12 February 2026 | D'Inca, Rodolphe |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.....................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 20 5545

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2026

| Patent document cited in search report | Publication date | Patent family member(s) | Publication date |
|---|---|---|---|
| KR 102541059 B1 | 13-06-2023 | NONE | |
| CN 201392338 Y | 27-01-2010 | NONE | |
| CN 105911003 A | 31-08-2016 | NONE | |
| US 6451613 B1 | 17-09-2002 | AU 9129301 A | 22-03-2002 |
| | | CA 2421577 A1 | 14-03-2002 |
| | | EP 1328804 A1 | 23-07-2003 |
| | | JP 2004508551 A | 18-03-2004 |
| | | KR 20030036759 A | 09-05-2003 |
| | | US 6451613 B1 | 17-09-2002 |
| | | US 2003040122 A1 | 27-02-2003 |
| | | WO 0221123 A1 | 14-03-2002 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5132094 A
- US 9791430 B
- US 5275957 A
- US 8618820 B